# EUROPÄISCHE PATENTSCHRIFT

(11) **EP 3 880 276 B1**
(45) Veröffentlichungstag und Bekanntmachung des Hinweises auf die Patenterteilung: **15.01.2025**
(21) Anmeldenummer: 19802161.0
(22) Anmeldetag: 11.11.2019
(51) Int. Cl.: A61M 5/30

(54) **NADELFREIES INJEKTIONSGERÄT ZUR NADELFREIEN INJEKTION EINER WIRKSTOFFHALTIGEN LÖSUNG**
NEEDLE-FREE INJECTION APPLIANCE FOR NEEDLE-FREE INJECTION OF AN ACTIVE SUBSTANCE SOLUTION
INSTRUMENT D'INJECTION EXEMPT D'AIGUILLE UTILISÉ POUR RÉALISER UNE INJECTION SANS AIGUILLE D'UNE SOLUTION À TENEUR EN PRINCIPE ACTIF

(30) Priorität: 12.11.2018 DE 102018128273
(43) Veröffentlichungstag der Anmeldung: 22.09.2021
(73) Patentinhaber: Predescu-Melzer, Alina, 8805 Richterswil (CH)
(72) Erfinder: Predescu-Melzer, Alina, 8805 Richterswil (CH)
(74) Vertreter: Kleine, Hubertus
(86) Internationale Anmeldenummer: PCT/EP2019/080886
(87) Internationale Veröffentlichungsnummer: WO 2020/099331

(56) Entgegenhaltungen:
- EP-A2- 0 295 917
- US-A- 3 688 765
- US-A- 4 941 880
- US-B1- 6 264 629

## Beschreibung

Die vorliegende Erfindung betrifft ein nadelfreies Injektionsgerät nach dem Oberbegriff des Anspruchs 1.

Nadelfreie Injektionsgeräte sind aus dem Stand der Technik bekannt. Dabei wird ein Druck auf die wirkstoffhaltige Lösung ausgeübt, welche derart groß ist, dass sie die Hautoberfläche durchdringt.

Bei der Wahl, wie der Druck aufgebaut wird sind verschiedene Varianten bekannt. So unterscheidet man zwischen einem federbetriebenen Spannmechanismus welcher einen Federdruck auf einen Kolben ausübt und zwischen einem pneumatischen Mechanismus, welcher mit einem Gasdruck arbeitet.

Weiterhin findet man unter der Internetseite http://news.mit.edu/2012/needleless-injections-0524 die Beschreibung einer nadellosen Injektionsvorrichtung, welche mit magnetischer Abstoßung arbeitet.

Im Bereich der federbetriebenen Spannmechanismen sind bereits Injektionsgeräte bekannt, welche einen Hebel zum Spannen eines Federmechanismus aufweisen. Ein Spannen eines Antriebsmechanismus ist bei einem pneumatischen Antrieb hingegen unnötig, da bereits auf ein Druck aufgebaut ist, welcher lediglich freigegeben werden muss.

In der US 6,264,629 B1 wird u.a. in Fig. 5 eine Injektionsgerät mit pneumatischem Mechanismus vorgestellt. Anhand der Kraftlinien kann man erkennen, dass der Betätigungsdruck auf eine Kappe axial zur Geräteachse her eingebracht wird.

Ein solcher Krafteintrag aus axialer Richtung führte zu einem unerwünschten zusätzlichen axialen Druck auf Injektorgerätes somit auch auf die Haut und die Injektionsstelle. Dies kann beispielsweise bei entzündeten Stellen zu unnötigen Schmerzen oder zu einer Ausbereitung des Entzündungsherdes führen. Zudem muss ein erheblicher Druck aufgebracht werden, um die Gaskartusche auszulösen. Der Druck in der Gaskartusche muss für die Anwendung relativ hoch sein, so dass die Verschlussvorrichtung, welche vorab zerstört werden muss, relativ stabil ist.

Als weiterer Stand der Technik wird die US 3 688 765 A genannt. Hier wird eine stiftartige gasbetriebene Injektionsspritze offenbart, in welchem eine federbelastete Nadel angeordnet ist, welches durch Betätigung eines Sperrhebels durch die Feder in eine feststehende Gaskartusche gepresst wird.

Die US 4 941 880 A offenbart eine gasbetriebene Injektionsspritze in welcher ein Dorn, welcher linear beweglich im Spritzengehäuse angeordnet ist, über einen Hebelmechanismus in eine Gaskartusche gepresst wird.

Die EP 0 295 917 A2 offenbart einen zweistufigen Betätigungsmechanismus einer gasbetriebenen Injektionsspritze. In einem ersten Schritt wird eine Gaskartusche auf eine Kanüle gepresst. Dies erfolgt durch Betätigung einer endständigen Flügelschraube. Entsprechend abgedichtet müssen die Übergänge zur Kanüle sein. Ein Hebelmechanismus verschließt (ebenfalls gasdicht) die Öffnung der Kanüle. In einem zweiten Schritt erfolgt die Freisetzung eines Gases aus der Kanülenöffnung durch Verkippen des Hebelelements durch Betätigen eines seitlichen Knopfes.

Aufgabe der vorliegenden Erfindung ist es angesichts des vorgenannten Standes der Technik ein kostengünstig herstellbares und einfach bedienbares Injektionsgerät bereitzustellen.

Die vorliegende Erfindung löst diese Aufgabe durch das Bereitstellen eines nadellosen Injektionsgerätes mit den Merkmalen des Anspruchs 1.

Ein erfindungsgemäßes nadelfreies Injektionsgerät dient zur nadelfreien Injektion einer wirkstoffhaltigen Lösung durch die Hautoberfläche in ein darunter angeordnetes subkutanes Gewebe.

Das Injektionsgerät weist ein Injektorgehäuse und eine Gaskartusche auf. Weiterhin weist das Injektionsgerät einen Kolben, beispielsweise einen stangenförmigen Spritzenkolben, auf, welcher durch einen Druck aufgrund einer Gasfreisetzung von Gas aus der Gaskartusche bewegbar, insbesondere linear bewegbar, im Injektorgehäuse angeordnet ist. Hierfür kann das Injektorgehäuse einen Kanal aufweisen welcher zur Führung des Kolbens dient. Der Kolben wirkt auf die wirkstoffhaltige Lösung welche in einem Kanal angeordnet sein kann, welcher sich linear in Bewegungsrichtung des Kolbens im Gehäuse erstreckt.

Durch die Bewegbarkeit des Kolbens wird der Druck auf die wirkstoffhaltige Lösung übertragen. Die Lösung wird durch die Druckübertragung über eine Ausgabeöffnung im nadelfreien Injektionsgerät beim Aufsetzen auf die Haut an das subkutane Gewebe abgegeben.

Das nadelfreie Injektionsgerät weist erfindungsgemäß zudem ein Hebelelement auf, bei dessen Betätigung die Gaskartusche zur Gasfreisetzung auf eine innerhalb des Injektorgehäuses angeordneten und zur Gaskartusche hin spitz-zulaufende Düse gepresst wird.

Dadurch wird Gas zur Bewegung des Kolbens unter Injektion der wirkstoffhaltigen Lösung freisetzt. Das Hebelelement ermöglicht eine einfachere und kraftverringerte Betätigbarkeit des erfindungsgemäßen Injektorgerätes.

Vorteilhafte Ausgestaltungsvarianten des Injektionsgeräts sind Gegenstand der Unteransprüche.

Das Hebelelement kann vorteilhaft zwei- oder mehrteilig mit mehreren Hebeln aufgebaut sein. Die jeweiligen Hebel sind dabei Teile des Hebelelements.

Zumindest ein Hebel des Hebelelements ist derart gelagert, dass das Hebelelement zur Gasfreisetzung zumindest bereichsweise, vorzugsweise in einer Richtung die parallel zur Gehäuseachse des Injektorgehäuses verläuft, auf die Gaskartusche drückt.

Das Hebelelement, insbesondere ein Hebel des Hebelelements, kann vorzugsweise zwei Schenkel aufweisen, welche in einem derartigen Winkel zueinander stehen, dass eine Kraftumlenkung von einer Betätigungskraft in radialer Richtung zu einer Gehäuseachse C des Injektorgehäuses in eine axial-gerichtete Kraft erfolgt.

Dabei muss die Kraftumlenkung nicht zwingend 90° betragen. Bevorzugt kann die Kraftumlenkung vorzugsweise um zumindest 70°, besonders bevorzugt um 80 bis 100°, erfolgen.

Die Gaskartusche kann derart befüllt sein, dass Sie ein Gasdruck von zumindest 100 ATM, vorzugsweise mehr als 180 ATM auf den Kolben freisetzt.

Das nadelfreie Injektionsgerät kann einen mit der wirkstoffhaltigen Lösung befüllten Kanal aufweisen mit der daran endständig angeordneten Ausgabeöffnung, wobei die Ausgabeöffnung einen Durchmesser aufweist, welcher zumindest viermal, vorzugsweise zehnmal geringer ist als der Durchmesser des Kanals. Dadurch wird eine weitere Druckerhöhung vor der Injektion der Lösung erreicht.

Ein erster der zumindest zwei Hebel des Hebelelements kann außerhalb des Injektorgehäuses z.B. an dessen Außenumfang angeordnet sein und einen Kippwinkel von zumindest 3°, vorzugsweise 5 bis 20°, gegenüber dem Injektorgehäuse aufweisen. Dies ermöglicht eine hinreichend große Hebelwirkung zur Kraftübertragung.

Der erste Hebel des Hebelelements kann eine im Wesentlichen lineare Form aufweisen, welche in Form einer Wippe um eine Kippachse gekippt werden kann, wobei ein erster Hebelarm zumindest viermal, vorzugsweise achtmal so lang ist wie ein zweiter Hebelarm, welcher an einem zweiten Hebel angreift.

Der zweite Hebel des Hebelelements kann eine gekrümmte Form , insbesondere L-förmige Form aufweist, wobei ein erster Hebelarm des zweiten Hebels, an welchem der erste Hebel angreift, vorzugsweise doppelt so lang ist wie ein zweiter Hebelarm zum axialen Druckeintrag auf die Gaskartusche.

Das Injektionsgerät, insbesondere das Injektorgehäuse, kann mehrteilig aufgebaut sein, wobei die jeweiligen Teile miteinander lösbar verbunden sind. Es kann eine pneumatische Antriebseinheit mit der Gaskartusche aufweisen, sowie eine Kraftübertragungseinheit zur Übertragung des Drucks auf die Lösung. Die Kraftübertragung weist den linear im Injektorgehäuse beweglich angeordneten Kolben auf. Schließlich kann das Injektionsgerät auch eine Wirkstoffkammer aufweisen mit dem Kanal zur Aufbewahrung und zum Freisetzen der wirkstoffhaltigen Lösung in das subkutane Gewebe.

Das Injektionselement kann ein Sperrelement aufweisen, welcher das Hebelelement vor einer unbeabsichtigten Nutzung sperrt und dessen Deaktivierung z.B. dessen Entfernung eine Kippbewegung des Hebelelements freigibt.

Das Injektorgehäuse und das Hebelelement können kostengünstig aus Kunststoff gefertigt sein.

Das Hebelelement bei dessen Betätigung unmittelbar, also ohne weitere kraftübertragende Elemente wie Federn oder dergleichen, eine Kraft auf die Gaskartusche übertragen.

Durch seinen einfachen Aufbau, der Möglichkeit der Verwendung von kostengünstigen Bauteilen und der geringen Anzahl an Bauteilen eignet sich das Injektionsgerät für den Einmalgebrauch.

Nachfolgend wird die Erfindung anhand eines Ausführungsbeispiels unter Zuhilfenahme der beiliegenden Figuren näher erläutert. Es zeigen
- Fig. 1: schematische Schnittansicht eines ersten erfindungsgemäßen Injektionsgeräts;
- Fig. 2: Draufsicht auf das erfindungsgemäße Injektionsgerät;
- Fig. 3: Schnittansicht auf das erfindungsgemäße Injektionsgerät auf Höhe der Schwenkachse eines Hebelelements; und
- Fig. 4: schematische Schnittansicht eines zweiten erfindungsgemäßen Injektionsgeräts,

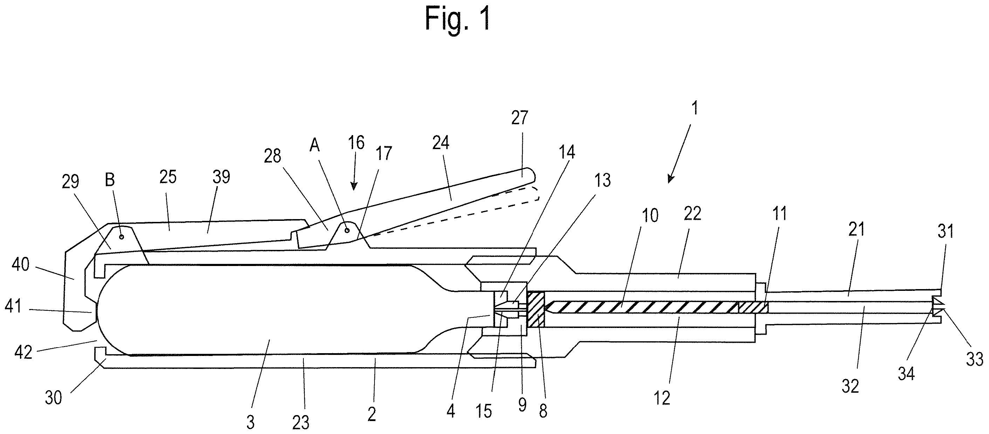

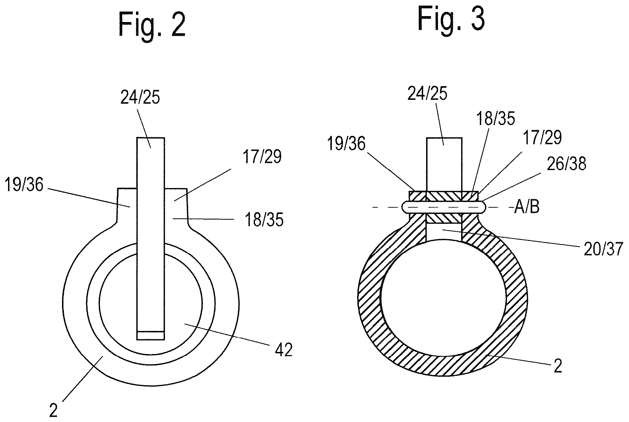

Fig. 1-3 zeigt eine Ausführungsvariante eines erfindungsgemäßen Injektionsgeräts 1.

Das Injektionsgerät 1 dient der nadelfreien Injektion einer wirkstoffhaltigen Lösung in ein subkutanes Gewebe. Das Injektionsgerät 1 weist ein Injektorgehäuse 2, welches in Fall der Fig. 1 in drei Sensorgehäuseabschnitte 21, 22 und 23 unterteilbar ist. Die Injektorgehäuseabschnitte 21, 22 und 23 können lösbar miteinander, z.B. durch eine Schraubverbindung, verbunden sein.

Ein erster Injektorgehäuseabschnitt 21 weist eine Stirnfläche 31 auf welche zum Kontakt mit der Haut vorgesehen ist. Der erste Injektorgehäuseabschnitt 21 ist als stiftförmige Hülse ausgebildet. Er umfasst ein Kanal 32 zur Leitung der Lösung zu einer Ausgabeöffnung 33 zur Abgabe der Lösung an die Haut. Der Kanal 32 weist im Bereich der Abgabeöffnung 33 eine Querschnittsverringerung 34 auf. Die Querschnittsverringerung 34 weist einen Durchmesser auf, welcher zumindest zweimal geringer ist als der Durchmesser im übrigen Bereich des Kanals 32, vorzugsweise sogar um mehr als zehnmal geringer als der Durchmesser des Kanals 32. Die Querschnittsverringerung ermöglicht eine zusätzliche Druckerhöhung auf die abgegebene Lösung vor deren Auftreffen auf die Haut.

Im Initialzustand kann innerhalb des Kanals 32 die zu injizierende Lösung angeordnet sein. Die Länge des Kanals 32 kann je nach Dosierung der Menge an Lösung variieren. Dadurch ist es möglich den Kanal vollständig zu befüllen und endständig mit einer Folie oder dergleichen zu bedecken. Allerdings kann auch die Befüllung des Kanals 32 und die Zusammensetzung zum gesamten Injektionsgerät 1 auch schon werksseitig erfolgen.

In den Kanal 32 ist ein stangenförmiger Spritzenkolben 10 einführbar. Der Spritzenkolben 10 weist endständig ein Dichtaufsatz 11 auf, damit keine Lösung randseitig zum Spritzenkolben 10 ausweichen kann, was zu einer Druckverringerung auf die restliche Lösung im Kanal 32 führen kann.

An der Schnittstelle zwischen dem ersten Injektorgehäuseabschnitt 21 und dem sich daran anschließenden zweiten Injektorgehäuseabschnitt 22 ist eine Dichtung, z.B. ein Dichtring angeordnet, durch welche der stabförmige Spritzenkolben 10 beweglich geführt ist. Der zweite Injektorgehäuseabschnitt weist einen Kanal 12 zur Führung des stabförmigen Spritzenkolbens 10 und eines endständig-angeordneten und fest mit dem Spritzenkolben 10 verbundenen Dichtelements 8 auf. Aufgrund der Dimensionierung des Dichtelements 8 weist dieser Kanal 12 einen größeren Durchmesser auf als der Kanal 32 des ersten Injektorgehäuseabschnitts 21. Endständig am Kanal 12 ist ein Einsatz 9 mit einer zentralen Düse 13 angeordnet, welche eine Gaseinleitung in den Kanal 12 erlaubt und dort einen Druck auf das Dichtelement 8 ausübt, welcher den Spritzkolben 10 in eine Linearbewegung in Richtung der Abgabeöffnung 33 versetzt. Die Düse 13 verjüngt sich in entgegengesetzter Richtung zu der vorgenannten Linearbewegung und bildet eine Spitze 14 mit einer Gaseinlassöffnung.15, welche eine Zerstörung der Gaskartusche im Bereich eines Kartuschenverschlusses 4 durch Durchstechen des Verschlusses ermöglicht und ein Einleiten des freigesetzten Gases in den Kanal 12.

Der dritte Injektorgehäuseabschnitt weist einen Aufnahmeraum für eine Gaskartusche auf. Im Konkreten Fall der Fig. 1 wurde eine sogenannte 12 Gramm Gaskartusche 3 genutzt. Gaskartuschen 3 weisen einen mechanisch-zerstörbaren Kartuschenverschluss 4 auf. Im Fall des Ausführungsbeispiels der Fig. 1 kann es sich um einen durchstechbaren Kartuschenverschluss handeln. Selbstverständlich muss der Kartuschenverschluss hinreichend stabil sein, um den Gasdruck innerhalb der Gaskartusche zurückzuhalten. Daher ist bei pneumatischen betriebenen Injektionsgeräten nicht immer eine einfache Bedienung möglich, sondern es muss zur Zerstörung des Kartuschenverschlusses ein erheblicher Kraftaufwand betrieben werden.

Zusätzliche kraftunterstützende Bauteile, z.B. Federn und/oder Kappen, haben das Injektionsgerät meist derart verteuert, dass eine Verwendung als Einweg-Injektionsgerät aus Kostengründen nicht möglich war.

Die vorliegende Erfindung bietet zur Aktivierung der Gaskartusche 3 eine wesentlich einfachere und im Operationsbetrieb einfach handhabbare Lösung durch ein zweiteiliges Hebelelement 16. Das Hebelelement 16 ist gut zugänglich außenseitig am Injektorgehäuse 2 angeordnet. Das Hebelelement weist einen ersten im Wesentlichen linearen ersten Hebel 24 und einen zweiten L-förmigen Hebel 25 auf, welche miteinander wechselwirken.

Zur Anordnung des ersten Hebels 24 steht von dem im Wesentlichen zylindrischen dritten Injektorgehäuseabschnitt 23 eine erste Hebellagerung 17 hervor. Die Hebellagerung 17 umfasst zwei Vorsprünge 18, 19 und einen Spalt 20 in welchen der Hebel 24 zumindest bereichsweise einführbar und mittels eines Achsbolzens 26, welcher in Ausnehmungen in den beiden Vorsprüngen 18 und 19 angeordnet ist, kippbar gelagert. Dieser erste Hebel 24 wird nachfolgend auch als Kipphebel bezeichnet. Der Achsbolzen 26 definiert einen Kippachse A, um welche der Hebel 24 eine Kippbewegung ausführt. Dabei wird der Hebel 24 durch die Kippachse in einen ersten und einen zweiten Hebelarm 27, 28 aufgeteilt, wobei der erste Hebelarm 27 zumindest viermal, vorzugsweise zumindest achtmal so lang ist wie der zweite Hebelarm 28.

Bei deaktivierter befüllter Gaskartusche 3 ist der erste Hebelarm 27 maximal von der Wandung des Injektorgehäuses 2 entfernt und der zweite kürzere Hebelarm 28 liegt auf der Wandung des Injektorgehäuses 2 auf. Der Hebelarm kann dabei ausgehend von der Kippachse A gegenüber dem Sensorgehäuse in einem Winkel von vorzugsweise mehr als 3 °, beispielsweise 5 bis 20°, verlaufen.

Zur Anordnung des zweiten L-förmigen Hebels 25 steht von dem im Wesentlichen zylindrischen dritten Injektorgehäuseabschnitt 23 eine zweite Hebellagerung 29 hervor. Die zweite Hebellagerung 29 ist gegenüber der ersten Hebellagerung 17 zu einem der Stirnfläche 31 entgegengesetzten Endbereich 30 des Injektorgerätes 1 angeordnet. Analog zur ersten Hebelhalterung 17 weist auch die zweite Hebelhalterung 29 zwei Vorsprünge 35 und 36 und einen Spalt 37 auf, in welchen der Hebel 25 zumindest bereichsweise einführbar und mittels eines Achsbolzens 38, welcher in Ausnehmungen in den beiden Vorsprüngen 35 und 36 angeordnet ist, verschwenkbar gelagert. Dieser erste Hebel 25 wird nachfolgend auch als Schwenkhebel bezeichnet. Durch die Bewegung dieses Hebels erfolgt eine Kraftumlenkung einer Betätigungskraft von einer radialen Richtung gegenüber dem Injektorgehäuse 2 hin zu einer axialen Richtung, wo die Kraft endständig auf die Gaskartusche 2 drückt. Der Achsbolzen 38 definiert dabei eine Schwenkachse B

Der L-förmige Hebel weist dabei einen ersten Hebelarm 39, welcher vorzugsweise zumindest doppelt so lang ist wie der zweite Hebelarm 40. Endständig am ersten Hebelarm 39 greift der zweite Hebelarm 28 des ersten Hebels 24 an und drückt den ersten Hebelarm 39 in radialer Richtung vom Injektorgehäuse 2 wegdrückt.

Der zweite Hebelarm 40 ist bogenförmig ausgebildet, greift an einem zum Kartuschenverschluss entgegengesetzten Ende der Gaskartusche 3 und übt bei Betätigung des ersten Hebelarms 39 in der vorbeschriebenen Weise einen axialen Druck auf das Ende der Gaskartusche 3 auf.

Hierfür weist der Hebelarm 40 zur Kraftverstärkung einen zur Gaskartusche 3 hin hervorstehenden Vorsprung 41 auf, welcher durch eine endständige Öffnung 42 im Injektorgehäuse unmittelbar auf das Ende der Gaskartusche drücken kann.

Durch die Betätigung des Hebelelements 16 wird somit gezielt Kraft auf die Gaskartusche 3 übertragen, jedoch nur wenig axial-gerichtete Kraft auf das umgebende Injektorgehäuse 2. Durch die übertragenen Kraft wird die Gaskartusche auf die Düse 13 gepresst und ein Gasdruck auf den Spritzenkolben ausgeübt, welcher durch Linearbewegung im Kanal 12 und 32 einen hohen Druck auf die wirkstoffhaltige Lösung ausübt, so dass diese die Hautoberfläche durchdringt. Das dargestellte Injektionsgerät 1 zeichnet sich durch eine geringe Teileanzahl aus, welche in großer Stückzahl fertigbar sind. Dadurch ist das Injektionsgerät für den Einsatz als Einmal-Injektionsgerät, oft auch als Disposable Injektor bezeichnet, geeignet. Nachträgliche Sterilisationsmaßnahmen sind bei dieser Art von Injektionsgeräten zum einmaligen Gebrauch unnötig.

Abgesehen von den vorbeschriebenen Dichtelementen, dem Spritzenkolben, der Düse und der Gaskartusche können alle weiteren Teile, insbesondere das Injektorgehäuse 2 und das Hebelelement 16, aus Kunststoff gefertigt werden.

Als Gaskartusche 3 kann eine CO₂-Kartusche z.B. mit 12 g Gewicht eingesetzt werden. Diese Gaskartusche kann 200 Atm Druck im Bereich der Düse 13 freisetzen. Durch die Querschnittsverringerung 34 den endständigen Durchmesser im Bereich der Ausgabeöffnung 33 in einem bevorzugten Bereich von 0,16 bis 0,2 mm im Vergleich zum Kanal 32 mit einem Durchmesser von vorzugsweise zwischen 2 bis 4 mm, kann eine zusätzliche Druckerhöhung, vorzugsweise um das 10-fache, z.B. auf 3000 Atm erreicht werden.

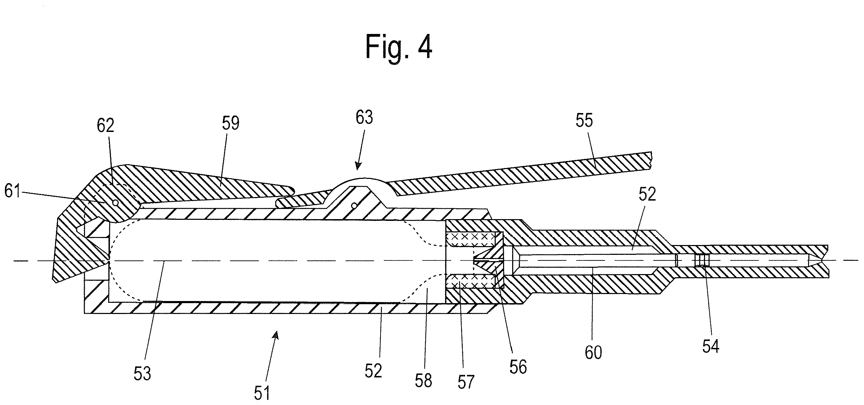

Fig. 4 zeigt eine zweite Ausführungsvariante eines erfindungsgemäßen Injektionsgerätes 51.

Dieses Injektionsgerät 51 weist, analog zur Variante der Fig. 1-3, ein Injektorgehäuse 52 mit einem Kartuschenlagerraum 58 auf und eine innerhalb dieses Kartuschenlagerraumes linear-verschieblich angeordnete Gaskartusche 53, welche durch ein mehrteiliges Hebelelement 63 betätigbar ist.

Das Injektorgehäuse weist endständig im Kartuschenlagerraum 58 einen Betätigungseinsatz mit einer spitz zulaufenden Düse 56 umfassend einen Dorn zum Durchstoßen des Kartuschenverschlusses der Gaskartusche 53 und mit einem Kanal zur Überleitung des Gases in einen Kolbenlagerraum 63, in welchem ein Kolben 60 linear verschieblich gelagert ist.

Die Düse 56 ist ein gesondertes Bauteil des Injektionsgerätes 51, welches über einen Einsatzhalter 57 mit einer Kartuschendichtung im restlichen Injektorgehäuse 52 gehalten wird.

Die Kartuschendichtung weist vorzugsweise eine zylindrische Form auf. Sie dient vorzugsweise zur Linearführung eines endständigen Fortsatzes der Gaskartusche 53.

Analog zu Fig. 1-3 weist auch das Injektionsgerät 51 eine Kombination aus zwei Hebeln 55 und 59 auf, welcher eine Krafteinleitung auf die Gaskartusche 53 analog zur ersten Ausführungsvariante ermöglicht.

Der Spritzenkolben 60 wirkt auf einen Dichtaufsatz 54, welcher in dieser Variante nicht mit dem Spritzenkolben 60 verbunden ist.

Das Injektionsgerät 51 weist endständig eine Ausgabeöffnung auf, welche in der Variante der Fig. 4 als Fortführung eines Kanal zur Führung des Kolbens 60 in seiner Vorwärtsbewegung ausgebildet und unter Querschnittsverengung spitz zulaufend ist.

Die Gaskartusche 53, z.B. als CO₂-Kartusche kann austauschbar in den Kartuschenlagerraum 58 eingesetzt werden.

Das pneumatisch-betätigte nadellose Injektionsgerät bzw. Spritze kann desinfiziert, versiegelt und gebrauchsfertig-befüllt als sogenannte Einmal-Anwendung (single use-application) ggf. verpackt ausgeliefert werden.

Nach der Entnahme des Injektionsgerätes 51 aus einer Verpackung, kann eine Drehsicherung 62 deaktiviert werden. Die Drehsicherung 62 kann z.B. ein Sicherungsstift sein, welcher verhindert, dass die Beweglichkeit des Hebels 55 und die Kippachse A oder des L-förmigen Hebels 59 die Schwenkachse B gegeben ist.

Nach dem Entfernen dieses Sicherungsstiftes kann die Kraftübertragung durch Betätigen des Hebels 55 auf die Gaskartusche 53 erfolgen. Zuvor wird die Spitze des Injektionsgerätes 51 auf den erwünschten Punkt auf der Haut aufgesetzt. Durch geringen Fingerdruck auf den Hebel 55 kann dieser um eine Strecke vor vorzugsweise 10-30 mm, vorzugsweise 15-20 mm, bewegt werden, bis es gegen das Injektorgehäuse 52 gepresst wird.

Dadurch wird die Linearbewegung der Gaskartusche 53 in Richtung des Dorns des Betätigungseinsatzes bewegt, so dass die Gaskartusche geöffnet wird und Gas über den Kanal des Betätigungseinsatzes Gas auf den Spritzenkolben 10 bzw. auf ein Dichtelement, welches endständig am Spritzenkolben 10 angeordnet ist, geleitet wird.

Die Kraftverstärkung auf die Gaskartusche 53 kann durch das Verhältnis der beiden Hebelarme 55 und 59 und deren geometrische Ausgestaltung und Lagerung um mehr als das 10-fache, sogar um das 13-fache, gegenüber eines direkten endständigen händischen Drückens auf den Kartuschenboden erhöht werden.

Dadurch wird eine leichtgängige Handhabung gewährleistet und eine Vermeidung von übermäßigen Druck auf die Injektionsstelle.

### Bezugszeichen

- 1, 51: Injektionsgerät
- 2, 52: Injektorgehäuse
- 3, 53: Gaskartusche
- 4: Kartuschenverschluss
- 8: Dichtelement
- 9: Einsatz
- 10, 60: Spritzenkolben
- 11, 54: Dichtaufsatz
- 12: Kanal
- 13, 56: Düse
- 14: Spitze
- 15: Gaseinlassöffnung
- 16, 63: Hebelelement
- 17: Hebellagerung
- 18: Vorsprung
- 19: Vorsprung
- 20: Spalt
- 21: Sensorgehäuseabschnitt/Injektorgehäuseabschnitte
- 22: Sensorgehäuseabschnitt/Injektorgehäuseabschnitte
- 23: Sensorgehäuseabschnitt/Injektorgehäuseabschnitte
- 24. 55: Hebel
- 25, 59: L-förmiger Hebel
- 26: Achsbolzen
- 27: Hebelarm
- 28: Hebelarm
- 29, 61: Hebellagerung
- 30: Endbereich
- 31: Stirnfläche
- 32: Kanal
- 33: Ausgabeöffnung
- 34: Querschnittsverringerung
- 35: Vorsprung
- 36: Vorsprung
- 37: Spalt
- 38: Achsbolzen
- 39: Hebelarm
- 40: Hebelarm
- 41: Vorsprung
- 42: Öffnung

- 57: Einsatzhalter mit Kartuschendichtung
- 58: Kartuschenlagerraum
- 62: Drehsicherung / Sicherungsstift
- 63: Kolbenlagerraum

- A: Kippachse
- B: Schwenkachse
- C: Gehäuseachse

## Patentansprüche

1. Nadelfreies Injektionsgerät (1, 51) zur nadelfreien Injektion einer wirkstoffhaltigen Lösung in ein subkutanes Gewebe, wobei das Injektionsgerät (1, 51) ein Injektorgehäuse (2, 52) und eine verschiebliche Gaskartusche (3, 53) aufweist und einen Kolben (10, 60), welcher durch einen Druck aufgrund einer Gasfreisetzung von Gas aus der Gaskartusche (3, 53) linear bewegbar im Injektorgehäuse (2, 52) angeordnet ist, wobei durch den Kolben (10, 60) der Druck auf die wirkstoffhaltige Lösung übertragbar ist, welche über eine Ausgabeöffnung (33) im nadelfreien Injektionsgeräts (1, 51) durch Aufsetzen auf die Haut an das subkutane Gewebe abgebbar ist,
**dadurch gekennzeichnet, dass** das nadelfreie Injektionsgerät (1, 51) ein Hebelelement (16, 63) aufweist, bei dessen Betätigung die Gaskartusche (3, 53) zur Gasfreisetzung auf eine innerhalb des Injektorgehäuses (2, 52) angeordnete und zur Gaskartusche (3, 53) hin spitz-zulaufende Düse (13, 56) gepresst wird und dadurch Gas zur Bewegung des Kolbens (10, 60) unter Injektion der wirkstoffhaltigen Lösung freisetzt,
**und dass** das Injektorgehäuse (2, 52) einen Kartuschenlagerraum (58) aufweist, wobei die Gaskartusche (53) innerhalb dieses Kartuschenlagerraumes (58) linear-verschieblich angeordnet ist.

2. Nadelfreies Injektionsgerät nach Anspruch 1, **dadurch gekennzeichnet, dass** das Hebelelement (16, 63) zwei- oder mehrteilig mit mehreren Hebeln (24, 25, 55, 59) aufgebaut ist, wobei zumindest ein Hebel (25, 59) des Hebelelements (16, 63) derart gelagert ist, dass das Hebelelement (16, 63) zur Gasfreisetzung auf die Gaskartusche (3, 53) drückt.

3. Nadelfreies Injektionsgerät nach Anspruch 1 oder 2, **dadurch gekennzeichnet, dass** das Hebelelement (16, 63) , insbesondere ein Hebel (25, 55) des Hebelelements (16, 63), zwei Schenkel aufweist, welche in einem derartigen Winkel zueinander stehen, dass eine Kraftumlenkung von einer Betätigungskraft in radialer Richtung zu einer Gehäuseachse (C) des Injektorgehäuses (1, 51) in eine axial-gerichtete Kraft erfolgt, wobei die Kraftumlenkung vorzugsweise um zumindest 70°, besonders bevorzugt um 80 bis 100°, erfolgt.

4. Nadelfreies Injektionsgerät nach einem der vorhergehenden Ansprüche,
**dadurch gekennzeichnet, dass** die Gaskartusche (3, 53) derart befüllt ist,
dass Sie ein Gasdruck von zumindest 100 ATM, vorzugsweise mehr als 180 ATM auf den Kolben (10, 60) freisetzt.

5. Nadelfreies Injektionsgerät nach einem der vorhergehenden Ansprüche,
**dadurch gekennzeichnet, dass** das nadelfreie Injektionsgerät (1, 51) einen mit der wirkstoffhaltigen Lösung befüllten Kanal (32) aufweist, welcher endständig die Ausgabeöffnung (33) aufweist, wobei die Ausgabeöffnung (33) einen Durchmesser aufweist, welcher zumindest viermal, vorzugsweise zehnmal geringer ist als der Durchmesser des Kanals (32).

6. Nadelfreies Injektionsgerät nach einem der vorhergehenden Ansprüche,
**dadurch gekennzeichnet, dass** ein erster Hebel (24, 55) des Hebelelements (16, 63) außerhalb des Injektorgehäuses (1, 51) mit einem Kippwinkel von zumindest 3°, vorzugsweise 5 bis 20°, gegenüber dem Injektorgehäuse (2, 52) ausgebildet ist.

7. Nadelfreies Injektionsgerät nach einem der vorhergehenden Ansprüche,
**dadurch gekennzeichnet, dass** der erste Hebel (24, 55) des Hebelelements (16, 63) eine im Wesentlichen lineare Form aufweist, welche in Form einer Wippe um eine Kippachse (A) gekippt werden kann, wobei ein erster Hebelarm (27) zumindest viermal, vorzugsweise achtmal so lang ist wie ein zweiter Hebelarm (28) , welcher an einem zweiten Hebel (25, 59) angreift.

8. Nadelfreies Injektionsgerät nach einem der vorhergehenden Ansprüche,
**dadurch gekennzeichnet, dass** das zweite Hebel (25, 59) des Hebelelements (16, 63) eine gekrümmte Form , insbesondere L-förmige Form aufweist, wobei ein erster Hebelarm (39) des zweiten Hebels (25, 59), an welchem der erste Hebel (24, 55) angreift, vorzugsweise doppelt so lang ist wie ein zweiter Hebelarm (40) zum axialen Druckeintrag auf die Gaskartusche (3, 53).

9. Nadelfreies Injektionsgerät nach einem der vorhergehenden Ansprüche,
**dadurch gekennzeichnet, dass** das Injektionsgerät (1, 51), insbesondere das Injektorgehäuse (2, 52), mehrteilig aufgebaut ist, wobei die jeweiligen Teil miteinander lösbar verbunden sind, umfassend eine pneumatische Antriebseinheit mit der Gaskartusche (3, 53), sowie eine Kraftübertragungseinheit mit dem linear im Injektorgehäuse (2, 52) beweglichen Kolben (10, 60), und eine Wirkstoffkammer mit dem Kanal (32) zur Aufbewahrung und zum Freisetzen der wirkstoffhaltigen Lösung in das subkutane Gewebe.

10. Nadelfreies Injektionsgerät nach einem der vorhergehenden Ansprüche,
**dadurch gekennzeichnet, dass** das Injektionsgerät (1, 51) ein Sperrelement (62) aufweist, dessen Deaktivierung eine Kippbewegung des Hebelelements (16, 63) freigibt.

11. Nadelfreies Injektionsgerät nach einem der vorhergehenden Ansprüche,
**dadurch gekennzeichnet, dass** das Injektorgehäuse (1, 51) und das Hebelelement (16, 63) aus Kunststoff gefertigt ist.

12. Nadelfreies Injektionsgerät nach einem der vorherigen Ansprüche, **dadurch gekennzeichnet, dass** das Hebelelement (16, 63) bei dessen Betätigung unmittelbar eine Kraft auf die Gaskartusche (3, 53) überträgt.

## Claims

1. Needle-free injection device (1, 51) for the needle-free injection of a solution containing an active agent into a subcutaneous tissue, wherein the injection device (1, 51) has an injector housing (2, 52) and a displaceable gas cartridge (3, 53) and a plunger (10, 60) which is arranged in the injector housing (2, 52) so as to be linearly movable by a pressure due to a release of gas from the gas cartridge (3, 53), wherein the pressure is transferable by the plunger (10, 60) to the solution containing the active agent which can be dispensed to the subcutaneous tissue via a dispensing opening (33) in the needle-free injection device (1, 51) by placing it on the skin,
**characterized in that** the needle-free injection device (1, 51) has a lever element (16, 63), during the operation of which the gas cartridge (3, 53) is pressed onto a nozzle (13, 56) arranged within the injector housing (2, 52) and tapering towards the gas cartridge (3, 53) for the release of gas and thereby releases gas for moving the plunger (10, 60) under injection of the solution containing the active agent, **and in that** the injector housing (2, 52) has a cartridge storage chamber (58), wherein the gas cartridge (53) is arranged in a linearly displaceable manner within this cartridge storage chamber (58).

2. Needle-free injection device according to claim 1, **characterized in that** the lever element (16, 63) is constructed in two or more parts with a plurality of levers (24, 25, 55, 59), wherein at least one lever (25, 59) of the lever element (16, 63) is mounted in such a way that the lever element (16, 63) presses on the gas cartridge (3, 53) to release the gas.

3. Needle-free injection device according to claim 1 or 2, **characterized in that** the lever element (16, 63), in particular a lever (25, 55) of the lever element (16, 63), has two legs which are at such an angle to one another that a force redirection takes place from an actuating force in the radial direction to a housing axis (C) of the injector housing (1, 51) into an axially directed force, wherein the force redirection preferably takes place by at least 70°, particularly preferably by 80 to 100°.

4. Needle-free injection device according to one of the preceding claims, **characterized in that** the gas cartridge (3, 53) is filled in such a way that it releases a gas pressure of at least 100 ATM, preferably more than 180 ATM, on the plunger (10, 60).

5. Needle-free injection device according to one of the preceding claims, **characterized in that** the needle-free injection device (1, 51) has a channel (32) which is filled with the solution containing the active agent and has the dispensing opening (33) at its end, wherein the dispensing opening (33) has a diameter which is at least four times, preferably ten times, smaller than the diameter of the channel (32).

6. Needle-free injection device according to one of the preceding claims, **characterized in that** a first lever (24, 55) of the lever element (16, 63) is formed outside the injector housing (1, 51) with a tilting angle of at least 3°, preferably 5 to 20°, with respect to the injector housing (2, 52).

7. Needle-free injection device according to one of the preceding claims, **characterized in that** the first lever (24, 55) of the lever element (16, 63) has a substantially linear shape which can be tilted in the form of a rocker about a tilting axis (A), wherein a first lever arm (27) is at least four times, preferably eight times, as long as a second lever arm (28) which engages on a second lever (25, 59).

8. Needle-free injection device according to one of the preceding claims, **characterized in that** the second lever (25, 59) of the lever element (16, 63) has a curved shape, in particular an L-shaped shape, wherein a first lever arm (39) of the second lever (25, 59), on which the first lever (24, 55) engages, is preferably twice as long as a second lever arm (40) for the axial application of pressure to the gas cartridge (3, 53).

9. Needle-free injection device according to one of the preceding claims, **characterized in that** the injection device (1, 51), in particular the injector housing (2, 52), is of a multi-part construction, wherein the respective parts are releasably connected to one another, comprising a pneumatic drive unit with the gas cartridge (3, 53), as well as a force transmission unit with the plunger (10, 60) that can be moved linearly in the injector housing (2, 52), and an active substance chamber with the channel (32) for storing and releasing the solution containing the active substance into the subcutaneous tissue.

10. Needle-free injection device according to one of the preceding claims, **characterized in that** the injection device (1, 51) has a blocking element (62), the deactivation of which releases a tilting movement of the lever element (16, 63).

11. Needle-free injection device according to one of the preceding claims, **characterized in that** the injector housing (1, 51) and the lever element (16, 63) are made of plastic.

12. Needle-free injection device according to one of the preceding claims, **characterized in that** the lever element (16, 63) directly transfers a force to the gas cartridge (3, 53) when it is actuated.

## Revendications

1. Injecteur sans aiguille (1, 51) pour l'injection sans aiguille d'une solution contenant un principe actif dans un tissu sous-cutané, lequel injecteur (1, 51) comprend un corps d'injecteur (2, 52) et une cartouche de gaz (3, 53) coulissante et un piston (10, 60) qui est disposé dans le corps d'injecteur (2, 52) de façon à pouvoir être déplacé de façon linéaire par une pression résultant de la libération de gaz hors de la cartouche de gaz (3, 53), le piston (10, 60) pouvant transmettre la pression à la solution contenant le principe actif, qui peut être délivrée dans le tissu sous-cutané via une ouverture de sortie (33) de l'injecteur sans aiguille (1, 51) par l'application sur la peau, **caractérisé en ce que** l'injecteur sans aiguille (1, 51) comporte un élément formant levier (16, 63) dont l'actionnement presse la cartouche de gaz (3, 53) sur une buse (13, 56) disposée à l'intérieur du corps d'injecteur (2, 52) et diminuant en pointe vers la cartouche de gaz (3, 53) pour libérer du gaz, et du gaz est ainsi libéré pour déplacer le piston (10, 60) en injectant la solution contenant le principe actif
et **en ce que** le corps d'injecteur (2, 52) comporte un espace de stockage de la cartouche (58), la cartouche de gaz (53) étant disposée dans cet espace de stockage de la cartouche (58) avec possibilité de translation linéaire.

2. Injecteur sans aiguille selon la revendication 1, **caractérisé en ce que** l'élément formant levier (16, 63) est construit en deux ou plusieurs parties avec plusieurs leviers (24, 25, 55, 59), au moins un levier (25, 59) de l'élément formant levier (16, 63) étant supporté de telle manière que l'élément formant levier (16, 63) appuie sur la cartouche de gaz (3, 53) pour libérer le gaz.

3. Injecteur sans aiguille selon la revendication 1 ou 2, **caractérisé en ce que** l'élément formant levier (16, 63), en particulier un levier (25, 55) de l'élément formant levier (16, 63), comporte deux bras qui forment l'un avec l'autre un angle tel qu'il se produise une déviation d'une force d'actionnement dans le sens radial vers un axe de corps (C) du corps d'injecteur (1, 51) vers une force orientée dans le sens axial, la force étant de préférence déviée d'au moins 70°, de préférence de 80 à 100°.

4. Injecteur sans aiguille selon l'une des revendications précédentes, **caractérisé en ce que** la cartouche de gaz (3, 53) est remplie de telle manière qu'elle libère une pression de gaz de 100 atm, de préférence de plus de 180 atm, sur le piston (10, 60).

5. Injecteur sans aiguille selon l'une des revendications précédentes, **caractérisé en ce que** l'injecteur sans aiguille (1, 51) comporte un canal (32) rempli de la solution contenant un principe actif qui comporte l'ouverture de sortie (33) à son extrémité, l'ouverture de sortie (33) présentant un diamètre au moins quatre fois, de préférence dix fois plus petit que le diamètre du canal (32).

6. Injecteur sans aiguille selon l'une des revendications précédentes, **caractérisé en ce qu'**un premier levier (24, 55) de l'élément formant levier (16, 63) est formé à l'extérieur du corps d'injecteur (1, 51) avec un angle de pivotement d'au moins 3°, de préférence de 5 à 20°, par rapport au corps d'injecteur (2, 52).

7. Injecteur sans aiguille selon l'une des revendications précédentes, **caractérisé en ce que** le premier levier (24, 55) de l'élément formant levier (16, 63) présente une forme sensiblement linéaire, qui peut être pivotée autour d'un axe de pivotement (A) sous la forme d'une bascule, un premier bras de levier (27) étant au moins quatre fois, de préférence huit fois plus long qu'un deuxième bras de levier (28) qui se met en prise sur un deuxième levier (25, 59).

8. Injecteur sans aiguille selon l'une des revendications précédentes, **caractérisé en ce que** le deuxième levier (25, 59) de l'élément formant levier (16, 63) présente une forme coudée, en particulier une forme de L, un premier bras de levier (39) du deuxième levier (25, 59), sur lequel le premier levier (24, 55) se met en prise, étant au moins deux fois plus long qu'un deuxième bras de levier (40) destiné à exercer une pression axiale sur la cartouche de gaz (3, 53).

9. Injecteur sans aiguille selon l'une des revendications précédentes, **caractérisé en ce que** l'injecteur (1, 51), en particulier le corps d'injecteur (2, 52), est construit en plusieurs parties, les différentes parties étant reliées entre elles d'une façon qui peut être défaite, comprenant une unité d'entraînement pneumatique avec la cartouche de gaz (3, 53) et une unité de transmission de la force avec le piston (10, 60) mobile de façon linéaire dans le corps d'injecteur (2, 52) et une chambre à principe actif avec le canal (32) pour conserver la solution contenant un principe actif et la libérer dans le tissu sous-cutané.

10. Injecteur sans aiguille selon l'une des revendications précédentes, **caractérisé en ce que** l'injecteur (1, 51) comporte un élément de blocage (62) dont la désactivation débloque un mouvement pivotant de l'élément formant levier (16, 63).

11. Injecteur sans aiguille selon l'une des revendications précédentes, **caractérisé en ce que** le corps d'injecteur (1, 51) et l'élément formant levier (16, 63) sont faits de plastique.

12. Injecteur sans aiguille selon l'une des revendications précédentes, **caractérisé en ce que** l'élément formant levier (16, 63) transmet directement une force à la cartouche de gaz (3, 53) quand il est actionné.
